# EUROPEAN PATENT APPLICATION

(11) **EP 1 645 279 A1**
(43) Date of publication of application: **12.04.2006**
(21) Application number: 04748919.0
(22) Date of filing: 31.05.2004
(51) Int. Cl.: A61K 33/00, A61K 31/197, A61K 31/785, A61K 38/55, A61P 31/18

(54) **AGENT FOR INHIBITING MEMBRANE VIRUS REPRODUCTION, METHOD FOR THE PRODUCTION THEREOF, PHARMACEUTICAL COMPOSITION AND METHOD FOR INHIBITING VIRAL INFECTIONS**

(30) Priority: 23.06.2003 RU 2003118500
(71) Applicant: Rasnetsov, Lev Davidovich, Nizhny Novgorod, 603000 (RU)
(72) Inventor: RASNETSOV, Lev Davidovich, Nizhny Novgorod, 603000 (RU); SHVARTSAM, Iakov Yudelevitch, Nizhny Novgorod, 603105 (RU); LYALINA, Irina Konstantinovna, Nizhny Novgorod, 603140 (RU); RASNETSOVA, Betti Efimovna, Nizhny Novgorod, 603136 (RU)
(74) Representative: HOFFMANN EITLE
(86) International application number: PCT/RU2004/000208
(87) International publication number: WO 2004/112804

(57) **Abstract**

The present invention relates to the pharmaceutical industry and more particularly to the provision of an agent for inhibiting membrane virus replication. The object of the invention is to provide an agent based on fullerene polycarboxylic anions for suppressing the activity of membrane viruses in treating diseases caused by these viruses. For accomplishing said subject, there is proposed a group of inventions united by a common inventive concept, said group comprising a method for preparing compounds, studying the mechanisms of action, provision of pharmaceutical compositions, and developing methods of treating with their use. Said object t is accomplished by selecting such quantitative ratios of the components and reaction conditions, which ensure the preparation of polyaddition products. It has been established that in carrying out the synthesis the amount of the amino acid must exceed the amount of fullerene by more than 50 times. The product prepared by the proposed method has an unlimited solubility in water, required bioavailability, high efficiency of action on non-infected cells, low toxicity. The content of the main substance in the target product is at least 90%. The process is adaptable to streamline production and can be used in the pharmaceutical industry. Compositions of drugs for and methods of treating infectious diseases caused by human immunodeficiency virus (HIV), herpes simplex virus (HSC) and hepatitis C virus (HCV) have been developed.

## Description

### Field of the Art

The present invention relates to the pharmaceutical industry and more particularly to the provision of an agent for inhibiting membrane virus reproduction.

The present invention relates to compounds and pharmaceutically acceptable salts thereof which inhibit the replication of viruses having a glycolipid membrane, such as human immunodeficiency virus (HIV), herpes simplex virus (HSV), hepatitis C virus (HCV).

### Prior Art

At present investigations are under way for developing the therapy and methods of treating viral infections, especially those provoked by HSV, HCV and HIV/AIDS, and the AIDS-associated complex. AIDS patients whose immune system is disturbed suffer from numerous opportunistic infections caused by such pathogens as Pneumocystis carini and Candida albicans, HSV, cytomegalovirus (CMV), HCV or from some kinds of tumors (Kaposi's sarcoma), which become the direct cause of death. A method for treating AIDS is not known, and the current therapy in most cases is employed without sufficient proofs of its efficiency and has unfavorable side effects.

HIV is characterized by a high genetic and, consequently, antigenic variability. HIV strains obtained even from one and the same patient but at different stages of the patient's disease may differ in the antigenic properties and nucleotide sequences. A difference of strains in different climatogeographic zones. This complicates the chemotherapy, immunotherapy and vaccinal prevention of AIDS.

Most of the antiviral means employed so far for the treatment of HSV infections, including iodohydroxyuridine, cytosine, arabinose, adenine arabinoside and trifluoro-thymidine are substances which disturb the viral DNA synthesis. These substances influence also similar host cell functions, which involves cell toxicity problems and, as a consequence, makes their systematic use in humans impossible. At present the main drug for treating infections caused by HSV is acyclovir which has strong antiviral activity and low toxicity. However, its weak solubility and the appearance of drug-resistant viruses restrict the application of this drug.

No vaccine against hepatitis C has been developed so far, and interferon or interferon in combination with Virazole are mainly used as the suitable drugs. The treatment with these preparations is costly and insufficiently effective. It is known that only 25― 40% of HSV-infected individuals are responsive to the treatment with interferon.

The chemotherapy of AIDS is associated at present with the creation and use of reverse transcriptase inhibitors and also of HIV protease inhibitors. HIV reverse transcriptase (revertase) inhibitors are either of nucleoside nature: Zidovudine (AZT, Retrovir), Epivir 3TC (Lamivudine), Videx (ddl, Didianosine), Hivid (ddc, Zalcitabin), Zerit (d4T, Stavudine), Abacavir (ABC, Ziagen), Combivir (Zidovudine + Epivir), Trizivir (Abacavir + Epivir + Zxidovudine) or of non-nucleoside nature: Delavirdine (rescriptor), Nevirapine (Viramune), Efavirenz (Sustiva), or of nucleotide nature: Tenofovir, Viread. Russian-made preparation Phosphazide was registered in Russia. These preparations are toxic for a macroorganism as well, because they interfere in genomic structures. Reverse transcriptase synthesizes viral DNA throughout the period of the disease, and therefore it is necessary to use HIV revertase inhibitors for life.

At the present time HIV protease inhibitors are represented by several preparations: Saquinavir (Invvirase), Indinavir (Crixivan), Ritonavir (Norvir), Nelfinavir (Vira-cept), Amprenavir (Agenerase), Kaletra (Lopinavir + Ritonavir), HIV protease is responsible for the ripening (processing) of viral proteins. Disturbance of the glycoprotein processing leads to the inability of HIV varions to attach to the CD4 cell.

At presence there exists the term "third line therapy" for characterizing the treatment of HIV/AIDS patients in which the pathogen proved to be resistant to at least one drug from each class of the preparations, or in which the treatment with the use of two different schemes of therapy proved to be ineffective. Such highly active antiretroviral therapy (HAART) is also denoted by the terms "mega-HAART" or "giga-HAART". The third line therapy includes using simultaneously four antiretroviral preparations with different HIV reproduction blocking mechanisms: nucleoside and non-nucleoside reverse transcriptase inhibitors and protease inhibitors It is more complicated to prognosticate possible negative interactions of various drugs; as a result, the probability of the development of side reactions and complications sharply increases. In such cases constant monitoring of drug concentrations in blood serum is required, this being a costly procedure. The necessity of complementing the therapeutic course with preparations suppressing the activity of agents causing concomitant infections makes the process of treatment still more complicated.

The known medicinal preparations make it possible to control the course of the disease, but not to cure AIDS patients. The creation of drugs which can cure or at least provide better protection from the lethiferous virus is continued and is associated both with seeking new compounds capable of inhibiting the virus reproduction with the help of known mechanisms and with developing new approaches to solving the problem.

The range of preparations for treating HIV/AIDS patients is broadening. At present the following preparations are in the stage of clinical studies: Emtricitabine, DAPD (nucleoside analogs), Kapravirin, TMC-120 (non-nucleoside analogs). Attention is attracted also by such new protease inhibitors as Atazanavir (Erivada), Tipranavir, Mozenavir. Investigations are carried out with a view to creating preparations of absolutely new classes: interase inhibitors (S-1360), and also fusion inhibitors (Pentafuside (T-20 or Fuzeon)), T-1249, PRO-542, and chemokine receptor inhibitors (SCH-C and PRO-140). However, the results of tests are ambiguous. For instance, the SCH-C preparation caused an increase of the QT interval in the ECG of healthy individuals under test upon maximum dose administration, this being indicative of possible cardiological complications. Fusion inhibitors are polypeptides: T-20 consists of 36 natural amino acid fragments, T-1249 consists of 39 fragments. Use of these preparations is limited to intravenous administration; as a result of administering T-20, in some patients subcutaneous nodules were formed, occasionally subcutaneous infections and abscesses were noted.

High-polymer polyanionic natural compounds also deserve attention: peptidogly-cans, dextrans, polysaccharides, etc. These compounds are low-toxic and capable ob adsorbing viral particles and serve as a xenobiotic "trap" of HIV varions. It was shown that these substances inhibit the formation of syncytia, but direct effect of these drugs on the infectiousness of virus was not established (European Applications 04065512 and 0467185). As regards sulfonated polycarbamide, it was suggested (RU 2160746) that these substances with a molecular weight of from 2000 to 4000 suppress the HIV, HSV and HCV activity by the following mechanism: anionic groups of synthetic oligomers bind to the virus and/or cell membrane and thereby interrupt the virus ability to replication.

The majority of the known most dangerous viruses: HIV. HSV, HCV, CMV, influenza virus are typical representatives of membrane viruses. Infection of a host cell with membrane viruses is initially based on the interaction of various receptors on the surface of the host cell with virus glycoproteins. Than the viral and cell membranes fuse together and the virion contents flow into the host cell cytoplasm. An interference in this process might preclude the initial interaction of the virus and the host cell and their subsequent fusion, and also inhibit the formation of varions.

In WO 95/199491995 the possibility was shown for the first time of acting with one substance on two targets: on protease and HIV reverse transcriptase. In RU 2196602 a method for the simultaneous inhibition of HIV infection and CMV infection was proposed for the first time. The inhibition was carried out by the mechanism of blocking the active site on the molecule of protease and reverse transcriptase and of the late gB CMV human structural protein. In both patents fullerene derivatives were used.

Recent attention has been given to the biological activity of fullerenes in connection with the possibility of using them for combating viruses. The main obstacle on the path to creating medicinal preparations is connected with the insolubility of fullerenes in water, which hampers their direct administration into human organism.

Methods of preparing water-soluble forms of fullerene through the formation of an adduct with polyvinyl pyrrolidone are known Kiselev O.I. et al. // Mol. Materials, 1998, vol. 11, p. 41). Its effectiveness against influenza virus of type A and B is shown.

Also known is a method of preparing fullerenes, which comprises mixing fullerenes predissolved in an organic solvent with a polymeric matrix in chloroform, evaporating the mixture under vacuum until the solvents are completely removed, dissolving the resulting complex in a phosphate-salt buffer (pH 7.4―7.6), followed by ultrasonic treatment of the product (RU 2162819, 2001). Membrane cephalins are used as the water-soluble matrix.

The products prepared as a result of such modifications are unstable compounds, their aqueous solutions are suspensions, and this limits the possibility of their application and storage.

A promising trend is the provision of water-soluble fullerene derivatives by chemical synthesis. The prior art most relevant to the present invention are compounds and methods of preparing thereof described in patents WO 95/19949, RU 2196602, RU 2124022, US 6613771.

Known in the art is a compound containing a water-soluble fullerene derivative of the general formula C₆₀X = HOC(O)(CH₂)₂C(O)NH(CH₂)₂ (WO 95/19949, 1995, US 6613771, 2003). The substituents are any alkyl or aryl-alkyl substituents, in particular those which are substituted with nitrogen or oxygen, having from 1 to 20 carbon atoms. However, this compound has low water-solubility, equal to 1 mg/ml, and the method of preparing it is complicated.

In RU 2196602 a method is proposed for inhibiting HIV reproduction and CMV infection with the help of compounds based on the amino acid and dipeptide derivatives of fullerene. Sodium salts of fullerene-monoamino-caproic acid and fullerene-monoamino-butyric acids are used as the fullerene amino acid derivative.

The compound closest in terms of the technical essence and the technical result is the compound N-(monohydro)-fullerene-amino-caproic acid HC₆₀NH(CH₂)₅COOH (RU 2124022).

For preparing this compound, to a solution of fullerene in o-dichlorobenzene an aqueous solution of potassium salt of amino-caproic acid and 18-crown-6 are added. The reaction mass is stirred for 6―8 hours at 60°C. Then the solvents are distilled off, the residue is treated with a saturated solution potassium chloride, and the residue of the fullerene derivative is washed with water. The yield of the target product is quantitative. The obtained N-(monohydro)-fullerene-amino-caproic acid is soluble in dimethylsulfoxide, dimethylformamide, and pyridine.

This synthesis is disadvantageous in that the conditions of the reaction of fullerene C₆₀ and potassium salt of aminocaproic acid in a two-phase system lead to an increase of the process time; besides, 18-crown-6 used as a solubilizer is expensive.

The yield of the target product is small and does not exceed 5% of the weight of fullerene spent for the synthesis.

In all the patents described earlier products of the monoaddition of amino acids and peptides to fullerene. However, fullerenes have a large number of equivalent reaction centers along double bonds, this providing an opportunity of polyaddetion products to be formed.

### Disclosure of the Invention

It is an object of the present invention to provide an agent based on fullerene polycarboxylic anions for suppressing the activity of membrane viruses in treating diseases caused by these viruses.

For solving the posed problem there is proposed a group of inventions united by a common inventive concept, an agent which is a compound comprising a fullerene polycarboxylic anion, a method for the production thereof, a pharmaceutical composition comprising said agent, and a method for inhibiting the replication of membrane viruses

The essence of the invention consists in solving said problem and in an agent for inhibiting the reproduction of membrane viruses, which agent comprises a water-soluble compound of fullerene carboxylic anions of the general formula

C₆₀Hₙ[NH(CH₂)ₘC(O)O]ₙ,

where C₆₀ is the fullerene core,
NH(CH₂)ₘC(O)O⁻ is the aminocarboxylic anion,
m is an integer, preferably 3 and 5, most preferably 5,
n is an integer from 2 to 12, preferably from 4 to 6, most preferably 6.

The posed problem is also solved by the provision of a method for preparing an agent for inhibiting the reproduction of membrane viruses, wherein into a solution of fullerene in o-dichlorobenzene an amino acid is introduced in the form of a potassium or sodium salt, then a solubilizer is added, selected from the group of polyalkylene oxides: polyalkylene glycols with a molecular weight of from 150 to 400 or higher, and also dimethyl ethers of polyethylene glycols, or 18-crown-6, wherein the amount of the amino acid must exceed the amount of fullerene by more than 50 times, and the synthesis is carried out at a temperature of 60―80°C.

For solving the posed problem, there is also proposed a pharmaceutical composition for inhibiting the reproduction of membrane viruses, containing a water-soluble compound of fullerene polycarboxylic anions of the general formula

C₆₀Hₙ[NH(CH₂)ₘC(O)O⁻]ₙ,

where C₆₀ is the fullerene core,
NH(CH₂)ₘC(O)O⁻ is the aminocarboxylic anion,
m is an integer, preferably 3 and 5, most preferably 5,

n is an integer from 2 to 12, preferably from 4 to 6, most preferably 6 in an effective amount and pharmaceutically acceptable excipients.

The pharmaceutical composition for inhibiting the reproduction of membrane viruses is in the form of tablets, capsules, a solution for injections, suppositories.

In the method for inhibiting the reproduction of membrane viruses use is made of the above-characterized pharmaceutical composition for suppressing viruses in treating diseases caused by HIV/AIDS, herpes infections, viral hepatitis C.

The fullerene reaction with an amino acid salt in the medium of an organic solvent in the presence of a polyalkylene oxide gives water-soluble fullerene polycarboxylic anions of general formula (I)

C₆₀Hₙ[NH(CH₂)ₘC(O)O⁻]ₙ, (I)

where C₆₀ is the fullerene core,
NH(CH₂)ₘC(O)O⁻ is the aminocarboxylic anion,
m is an integer, preferably 5,
n is an integer from 2 to 12, preferably from 4 to 6.

For example:
C₆₀H₂[NH(CH₂)₃C(O)O⁻]₂ ― fullerene-di-amino-butyric anion
C₆₀H₂[NH(CH₂)₅C(O)O⁻]₂ ― fullerene-di-amino-caproic anion
C₆₀H₄[NH(CH₂)₅C(O)O⁻]₄ ― fullerene-tetra-amino-caproic anion
C₆₀H₆[NH(CH₂)₅C(O)O⁻]₆ ― fullerene-hexa-amino-caproic anion
C₆₀H₆[NH(CH₂)₇C(O)O⁻]₆ ― fullerene-hexa-8-amino-octanoic anion

The molecular weight is associated with the value n and m of the obtained compounds:
at n = 4 and m = 5 C₆₀H₄[NH(CH₂)₅C(O)O⁻]₄ (fullerene-tetra-amino-caproic anion) the molecular weight is 1240 g;
at n = 6 and m = 5 C₆₀H₆[NH(CH₂)₅C(O)O⁻]₆ (fullerene-hexa-amino-caproic anion) the molecular weight is 1500 g.

For preparing the agent, to a solution of fullerene in o-dichlorobenzene (toluene or any other acceptable organic solvent) an amino acid is added in the form of its (potassium or sodium) salt, then a solubilizer is added. The order of introducing the amino acid and solubilizer into the reaction medium is of no importance, they may be introduced in the form of a complex, after premixing thereof. As the solubilizer use is made of various polyalkylene oxides: polyethylene glycols with a molecular weight of from 150 to 400 and higher than 400 (for example, PEG-1500), and also polyethylene glycols having substituted terminal groups (for example, polyethylene glycol dimethyl ester with the molecular weight 500) or a cyclic structure (for example, 18-crown-6 for potassium salts).

The fullerene/amino acid ratio according to the present invention is increased more than 50-fold. When said ratio is smaller than 50-fold, the obtained compounds have smaller water solubility and high toxicity.

The optimal synthesis temperature is +(60―80)°C.

Conversion to the desired pharmaceutically acceptable salt, especially to sodium or potassium salt, can be accomplished by treating the acid with a suitable base. In particular, water-insoluble fullerene-polycarbloxylic acid is converted to more ;preferable pharmaceutically acceptable salts, such as sodium salt, which are soluble in water.

The yield of the target product is no less than 150% for the taken fullerene. The target product according to the present invention is characterized by constancy of the formulation, the content of the main substance in the target product being more than 90%,

Compounds of formula (I) are solid, dark-brown crystalline odorless substances, soluble in water in the salt form and insoluble in water in the acid form. Sodium salts of the claimed compounds upon dissolution in water form non-transparent solutions having saturated red-brown color. Sodium salts of compounds of formula (I) are soluble in glacial acetic acid, insoluble in 96% alcohol, o-dichlorobenzene, toluene and acetone. In the acid form the claimed compounds are readily soluble in DMSO (Example 1).

Compounds of formula (I) at 400°C and higher bum down completely, in contradistinction to fullerene, which melts at 420°C.

The authenticity of the clamed compounds of formula (I) has been confirmed by IR spectrometry in the 399―4000 cm⁻¹ region: the coincidence in the pattern and in the presence of absorption bands with fullerene was more than 90%; with amino acid, no less than 80%; the reproducibility in syntheses, no less than 80%. No such similarity was established for fullerene derivatives differing in amino acid radicals (Example 2).

A particular feature of the structure of the obtained compounds is the presence of several carboxyl groups in the molecule, which, depending on the pH of the medium, are either in the salt form or in the acid form, displaying buffer properties. The pH of transition of the claimed compounds to the dissolved state is 5.6―6.0.

The TLC was carried out on Merck 60F₂₅₄ silica gel. The best results for the separation of the components were obtained with the system of eluents : EtOH-benzene-H₂O 4:1:1.5. In the system there were found 3 spots with R_{f} equal to 0.82, 0.71 and 0.47 for the derivative of fullerene with aminocaproic acid, one spot for the derivative of fullerene with aminooctanoic acid (R_{f} = 0.82), and one spot for the derivative of fullerene with aminobutyric acid (R_{f} = 0.47). A sample with ninhydrin has shown the absence of compounds with a primary amino group in the product (Example 3).

¹H and ¹³C-NMR spectra of the solutions of compounds of formula (I) in deuterated solvents with different solvation capacities are recorded at 20°C on a WM-200 instrument with the working frequency 200.13 MHz for ¹H and 50.32 MHz for ¹³C

¹³C-NMR (δ, D₂O): 25.2 (CH₂CH₂C(O)O-), 25.4 (CH₂(CH₂)₂C(O)O-), 26.8 (CH₂C(O)O-), 69.5 (CH₂NH), 130―160 (C₆₀), 183.7 (C(O)O-).

¹H-NMR (δ, CD₃OD): 1.16 d (1H, J = 6.0 Hz, -NH-), 1.26; 1.45 and 1.65 (3 m, 1H, 3H respectively, -(CH₂)₃-), 2.18; 2.23; (2 s, 0.2H, -NH...), 2.34 t (2H, J = 7.2 Hz, -CH₂C(O)O-), 2.94 br t (0.4 H, J = 6.0 Hz, J = 1.8 Hz, -NCH₂-), 3.6 m (1H, J = 1.8 Hz, J = 6.0 Hz, C₆₀H).

¹H-NMR (δ, DMSO-d6): 1.02 d (0.4H, J = 6.0 Hz, -NH-), 1.22, 1.32 and 1.52 (3 m, 6H., -(CH₂)₃, 1.90; 2.08 (2 s, 0.3H each, -NH...), 2.20 t (2H, J = 7.2 Hz, -CH₂C(O)O-), 2.68 q (2H, -NCH₂-), 7.46 m; 8.17 s (0.5H each, C₆₀H), 12.08 br s (1H, -C(O)OH)).

¹H-NMR (δ, D₂O): 1.25 m, 1.49 m (2H, 4H respectively, J = 7.8 Hz, J = 6.9 Hz, -(CH₂)₃-), 1.88 d, (0.1H, J = 1.6 Hz, -NH...), 1.89 d (0.1H, J = 5.5 Hz, -NH...), 2.05 d (0.1H,, J = 1.6 Hz, -NH ...), 2.24 t (2H, J = 7.3 Hz, -CH₂C(O)O-),2.82 br t (1.5H, J = 1.6 Hz, J = 7.5 Hz, -NCH₂-), 3.49 m; (1H, J=5.5 Hz, C₆₀H).

The following properties of the proposed compounds are conditioned by the presence of the fullerene core in the molecule. The multitude of isolated multiple bonds allows one to regard fullerene as a polyolefin system. Addition via multiple bonds is most typical of fullerene. It easily adds nucleophiles and free radicals, which makes it possible to use such substances as antioxidants.

The technical result of the invention consists in the provision of a novel class of compounds: fullerene-carboxylic anions of general formula (I) by the nucleophilic addition of two and more amino acids to fullerene via several double bonds.

These compounds have new properties, different from fullerene, are noted for a better solubility in water than the analogs, whereby high efficiency of action on infected cells and low toxicity of the claimed compounds are provided.

A specific feature of the claimed compounds is a wide range of their antiviral activity with regard to various viruses pathogenic for man, including HIV, HSV, HCV.

It has been shown in experiments that with all the investigated methods of infecting cells under the action of compounds of formula (I) there takes placed inhibition of the virus-induced cytopathic action and lowering of the virus antigen level in the culture fluid. Thus, the preparation ― sodium salt of fullerene-polyamino-caproic acid in the concentration of 1 µg/ml provided full protection of reinoculated lymphoblastoid human cells MT-4 against viral cytopathic action (VCA) of HIV-1, taken in a dose of 100 TCD₅₀ to 7―10 days of follow-up after the infection of cell cultures. At the concentration of the preparation of 10 µg/ml, virus was not detected in the culture medium. In these concentrations and higher (to 100 µg/ml) no cytotoxic action of the preparation on cells was revealed. The effect of the claimed compounds in the studied concentrations of 1, 10 and 100 µg/ml on biosynthetic processes in the culture of lymphocyte cells has been shown to be absent during 96 hours by the electrophoresis of proteins and nucleic acids in 12.5% PAAG with silver nitrate staining, judging from the absence of changes of protein and nucleic profiles. In cells infected with human immunodeficiency virus, after 24―48 hours of cultivation, the profiles are weaker than in a normal culture and in the presence of the preparation. At the same time, in the presence of the claimed compounds in the studied concentrations (1 and 10 µg/ml) the profiles of virus-induced cells in the band intensity and spectrum correspond to the control culture. Other fullerene derivatives with aminobutyric and 8-aminooctanoic acids have demonstrated antiviral activity with respect to HIV-1 lower than with aminocaproic acid (Example 4).

The claimed compound: sodium salt of fullerene-polyamino-caproic acid in the concentration of 10 µg/ml provided full protection of the cells of the reinoculated culture of African green monkey kidney cells (VERO) and of human embryo fibroblasts (M-21) against the cytodestructive action of herpes simplex virus (HSV-1), taken in the dose of 100 TCD₅₀ 48 hours after the infection of the cell cultures. During the same period of time in the control infected cell cultures which had not undergone the action of the claimed compounds 100% death of the cells took place. The compounds of formula (I) ― fullerene derivatives with various amino acids ― are noted for antiviral activity with respect to HSV-1. (Example 5).

The antiviral activity of the compounds of formulas (I) was evaluated on an experimental model of HSV-induced infection in cultures of reinoculated cells of porcine embryo kidneys (SPEV). In our work we used a cytopathogenic strain of hepatitis C virus belonging to genotype 1b, in the dose of TCD₅₀. Thus, sodium salt of fullerene-polyamino-caproic acid in the concentration of100 µg/ml provided 100% viability of HCV-infected cells on the 7^{th} day after the infection. The obtained results have shown that the preparation in a concentration of up to 100 µg/ml does not have cytotoxic properties for SPEV cell cultures. At the same time, in the HCV-infected cell cultures cytopathogenic phenomena which affected 30―40% of the monolayer developed already by the 4^{th} day, and by the 7^{th} day, as a rule, all the HCV-infected cells died. The preparation has displayed similar activity also in the case when HCV-infected cells were treated with the preparation 24 hours after the infection. However, the preparation has maximum antiviral activity when administered simultaneously with the virus: the virus titers in cultures of SPEV cells lowered by 7.4 lg when the preparation concentration was 100 µg/ml and by 3.0 lg, when the preparation concentration was 50 µg/ml (Example 6).

An effect of inhibiting inhibition the proliferation of reinoculated cells of epithelial human carcinoma Hep2 by the action of the claimed compounds has been established. Thus, sodium salt of fullerene-polyamino-caproic acid in concentrations of 10, 50 and 100 µg/ml (most pronounced at 100 µg/ml) inhibits the formation of a monolayer of Hep2 cancer cells, which may be a reflect ion of its influence on the mitotic activity. Inhibiting of the proliferation is supported by a reduction of the monolayer formation rate, by a smaller content of proteins in samples containing the preparation , and by a reduction of the amount of nucleic acids therein. Data have been obtained, which testify to the absence of apoptosis induction under the influence of the preparation: DNA fragmentation is not recorded, no redistribution of the soluble and membrane mRNA forms, indicative of the possibility of Fas-dependent apoptosis, was detected (Example 7).

Pharmaceutical ready forms of the preparations of compounds of formula (I) can be prepared as a dosage form for oral or parenteral administration for the therapy or prevention of viral infections and conditions in which use of antioxidants and antidotes is indicated.

The compounds are mixed with conventional pharmaceutical carriers and used in the form of tablets, capsules, suppositories, ointments, solutions for injections, etc. The compositions comprising compounds of formula (I) contain approximately 0.1―90% by weight, preferably 0.5―10% by weight of an active compound. The compounds of the present invention can be administered orally, parenterally or rectally, including conventional non-toxic pharmaceutically acceptable carriers, stimulants and auxiliary agents. Such pharmaceutical compositions can be produced in the form of capsules or tablets for oral use, as sterile preparations for injections, or as suppositories. For oral use as capsules and tablets the compositions are prepared according to methods widely known in the field of preparing pharmaceutical formulations, and they may contain microcrystalline cellulose, starch to provide mass, magnesium stearate and lactose and/or other excipients, binders, expansion agents, disintegrators, diluents and lubricants, known in this field. Solutions for injections can be formed in accordance with the known methods, using non-toxic, parenterally applicable diluents or solvents, such as mannitol, 1,3-butanediol, water, Ringer's solution, isotonic solution of sodium chloride. In rectal administration in the form of suppositories such compositions can be prepared by mixing the drug with an equal amount of a non-irritating excipient, such as cocoa butter, synthetic glyceride esters or polyethylene glycols, that are solids at normal temperatures , but melt and/or dissolve in the rectal cavity with the drug release (Example 8).

The proposed compounds can be used for treating diseases caused by HIVE, HSV, HCV. Treating infectious diseases by acting with pharmaceutically acceptable doses of compounds of formula (I) is carried out affecting several viruses simultaneously and involves various virus replication stagers. It has been shown that the treatment is accompanied by lowering the stress effect to administering the preparation, by enhancing the antioxidant protection of the organism against infections. Organism intoxication is characteristic of the course of some viral infections and is responsible for the gravity of the disease. Calculated data have shown that dosage levels on the order of 0.1―250 or 2500 mg/day can be used for treating or preventing of the above-indicated conditions, oral dosages being 2―5 times higher. A particular level of dosages and the frequency of drug administration for each particular patient may vary and will depend on a large number of factors, including the activity of the chosen compound, its metabolic stability and time of action, the age of the patient, the bodyweight, general physical condition, sex, type and time of administration, rate of elimination, drug combinations (Example 9).

The compounds of formula (I) can be used together with other antiviral agents, immunomodulators. Anti-infective agents, or vaccines in various combinations with any pharmaceutical formulations intended for the treatment.

### Brief Description of the Drawings

Fig. 1. shows a spectrum of sodium salt of fullerene-polyamino-caproic acid recorded on a NICOLET "AVATAR 320-FT.IR" IR spectrometer, USA with NICOLET EZ OMNIC software, USA;
Fig. 2.1 confirms the authenticity of sodium salt of fullerene-polyamino-caproic acid by IR spectrometry techniques with the aid of NICOLET EZ OMNIC software, USA (the presence of a fullerene core);
Fig. 2.2 confirms the authenticity of sodium salt of fullerene-polyamino-caproic acid by IR spectrometry techniques with the aid of NICOLET EZ OMNIC software, USA (aminocaproic acid);
Fig. 3 shows the IR spectra and absorption bands of samples of sodium salt of fullerene-polyamino-caproic acid, recorded on the NICOLET "AVATAR 320-FT.IR" IR spectrometer, USA with NICOLET EZ OMNIC software, USA;
Fig. 4 shows the IR spectra and absorption bands of samples of sodium salt of fullerene-polyamino-caproic and fullerene-polyamino-butyric acids, recorded on the NICOLET "AVATAR 320-FT.IR" IR spectrometer, USA with NICOLET EZ OMNIC software, USA;
Fig. 5 shows the TLC of compounds of formula (I). There are shown three spots with R_{f} = 0.82, 0.71, 0.47 for samples of sodium salt of fullerene-polyamino-caproic acid (ACA-15, ACA-21, ACA-22 ― the numbers of syntheses are indicated), for sodium salt of fullerene-polyamino-butyric acid (ABA) ― R_{f} = 0.47, for sodium salt of fullerene-polyamino-octanoic acid (AOA) ― R_{f}= 0.82, fullerene remained at the start.
Fig. 6 shows the influence of compounds of formula (I) on biosynthetic processes in a culture of lymphoblastoid human cells infected with HIV1, during 96 hours. There are presented electrophoregrams of proteins:
   a) of non-infected culture;
   b) of HIV-1-infected culture;
   c) of non-infected cells in the presence of the preparation (sodium salt of fullerene-polyamino-caproic acid) in the concentration of 1 µg/ml;
   d) of HIV-1-infected cells with the preparation in the concentration of 1 µg/ml;
   e) of non-infected cells in the presence of the preparation in the concentration of 10 µg/ml;
   f) of HIV-infected cells with the preparation in the concentration of 10 µg/ml;

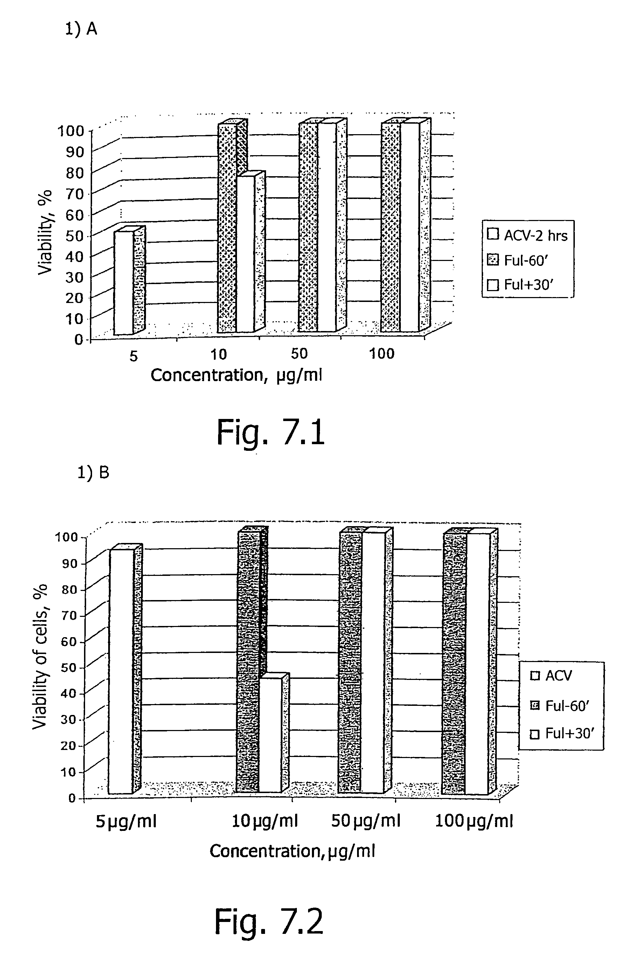

Fig. 7 illustrates comparative action of the compounds of formula (I) (fullerene) and acyclovir: protection of VERO cells (A) and M21 cells (B) from the cytopathic action of herpes virus type 1. The results obtained for sodium salt of fullerene-polyamino-caproic acid in concentrations of 10, 50, 100 µg/ml are used;

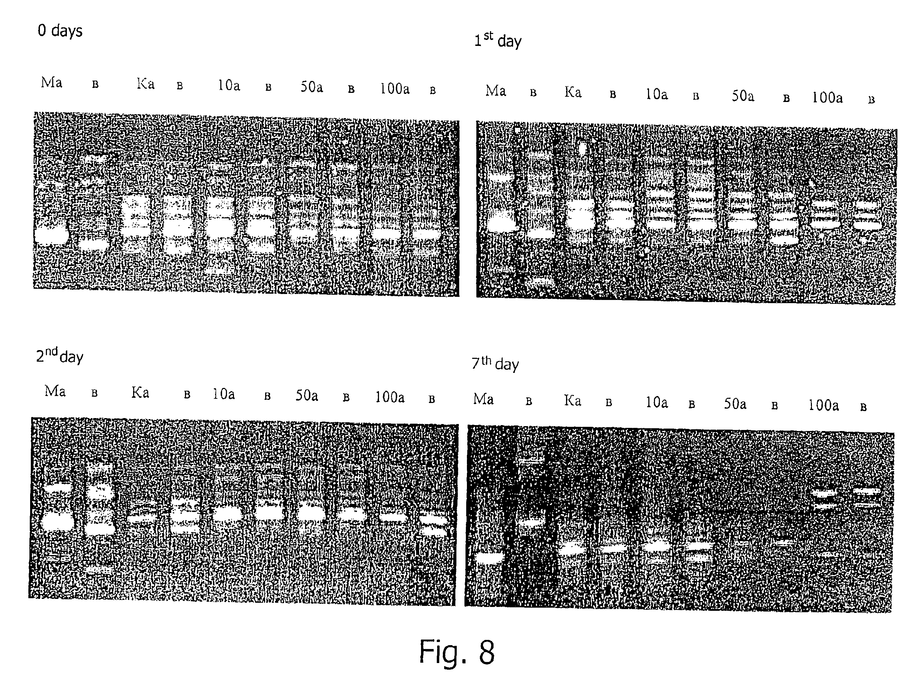

Fig. 8 shows a membrane form (a) and a soluble form (b) of Fas-antigen with sodium salt of fullerene-polyamino-caproic acid in concentrations of 10, 50, 100 µg/ml acting on an reinoculated culture of human epithelial carcinoma cells Hep 2.

### The Best Examples of Carrying out the Invention

**Example 1.** Synthesis. Characteristic of some compounds of formula (I).

A 2.5 g batch of fullerene is dissolved in o-dichlorobenzene (o-DCB), and half of the volume of PEG-500 and potassium salt of aminocaproic acid in the 1:1 mole ratio with PEG is added. The reaction mixture is maintained for at least 4 hours at the temperature of 70°C with stirring. The solvent is removed and the precipitate is dried till the odor of o-DCB disappears. The compounds in acidic form are prepared by adding about 120 ml of 8% hydrochloric acid, pH 5.0, and in salt form by dissolving in 0.1N sodium hydroxide, pH 7.0. The yield: 5.6 g of the product, 224% in terms of the taken fullerene.

A 360 mg batch of fullerene is dissolved in toluene. 16 g of potassium salt of aminobutyric acid and 50 g of PEG-500 are introduced into the solution. Then the procedure is continued as described above. The yield: 540g, 150% in terms of the taken fullerene.

The solubility of fullerene and of its derivatives is listed in Table 1..

**Example 2.** Spectrometric investigation in the IR spectral region of compounds of formula (I) in the form of acid or of their sodium salts was carried out in a disk with KBr. Fort this purpose 1 mg of the preliminarily dried preparation was mixed in a mortar with 150 mg of spectrometrically pure potassium bromide and the mixture was compacted at a pressure of 7.5―10 cm⁻¹ for 2―5 min.

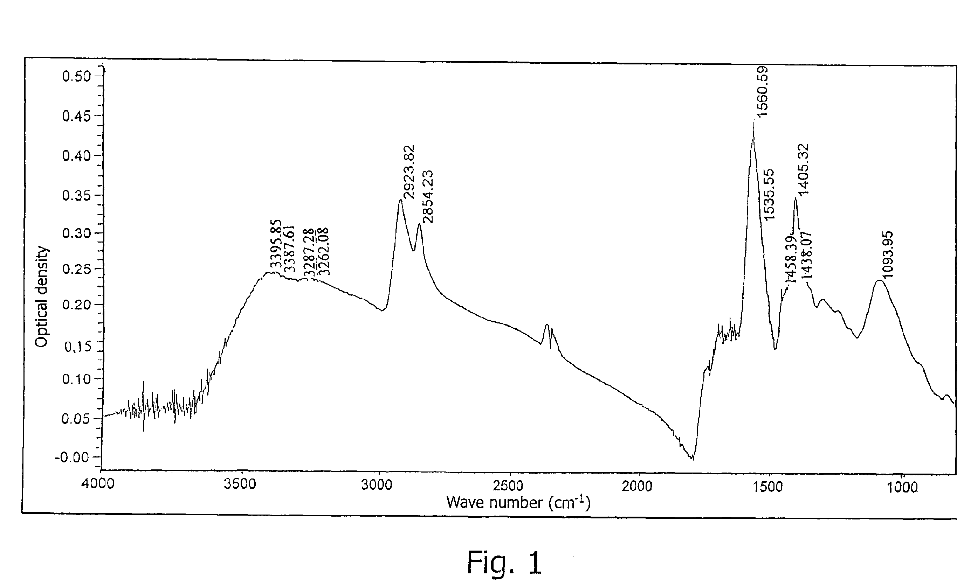

The spectrum of the obtained sample was recorded on a NICOLET "AVATAR 320-FT.IR" IR spectrometer, USA with NICOLET EZ OMNIC software, USA. Spectral parameters: resolution,. 4 cm⁻¹; format ― optical density; range, 399―4000 cm⁻¹; sampling frequency, 1.929 cm⁻¹. The spectrum was treated by correcting the H₂O/CO₂ line (Fig. 1). Concurrently under the same conditions the IR absorption spectra of fullerene and of the amino acids used in the synthesis were recorded.

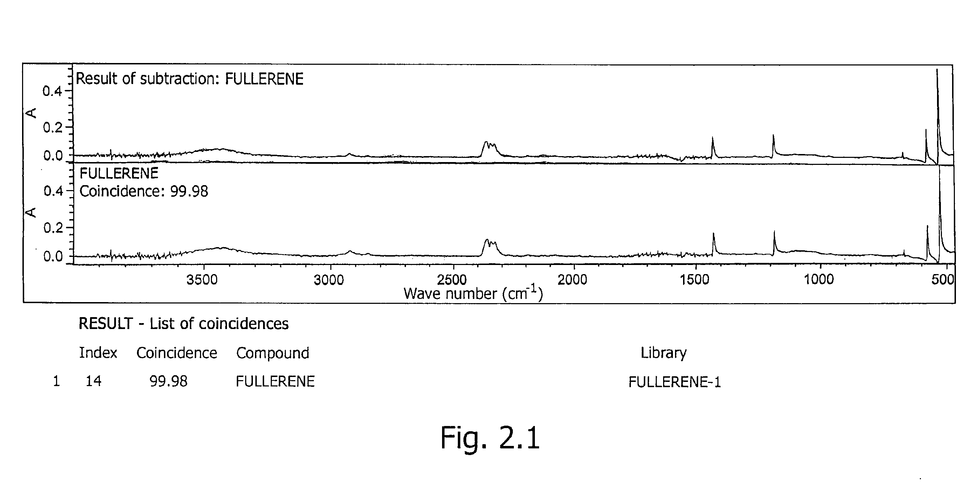

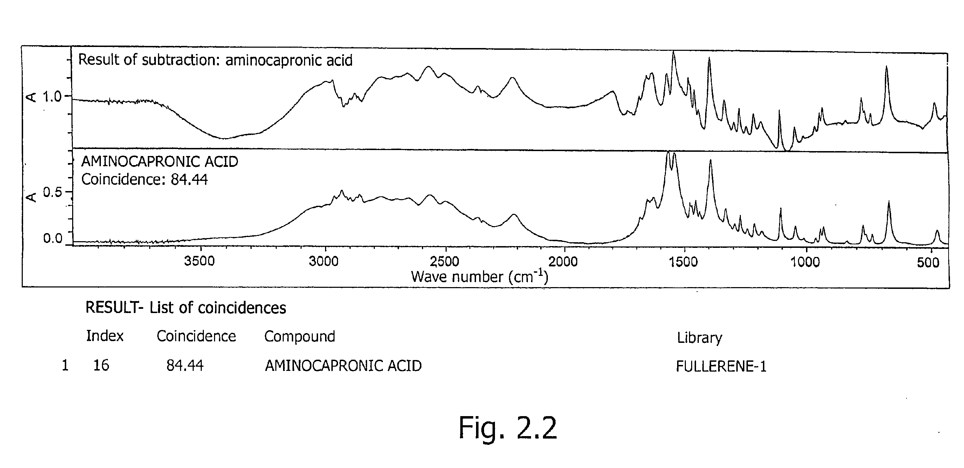

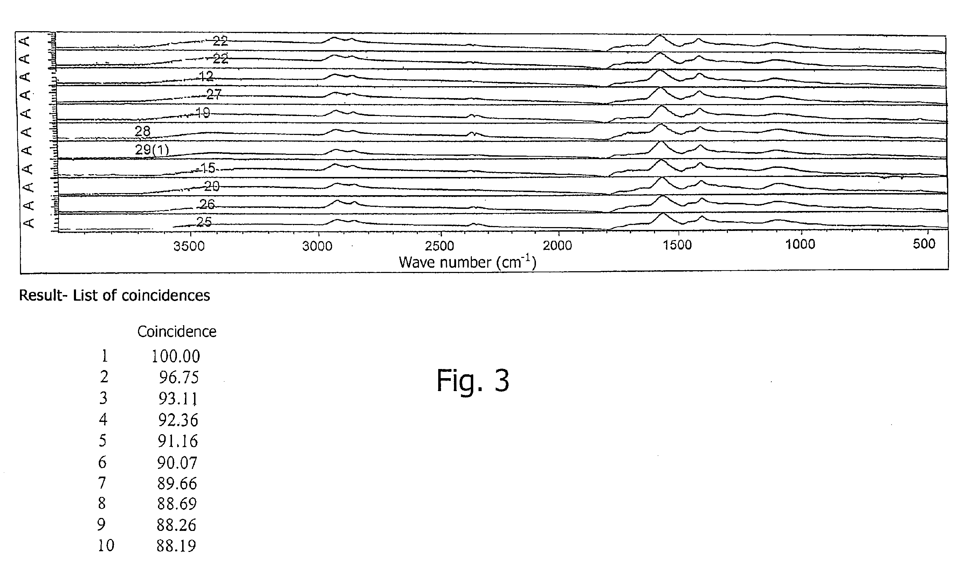

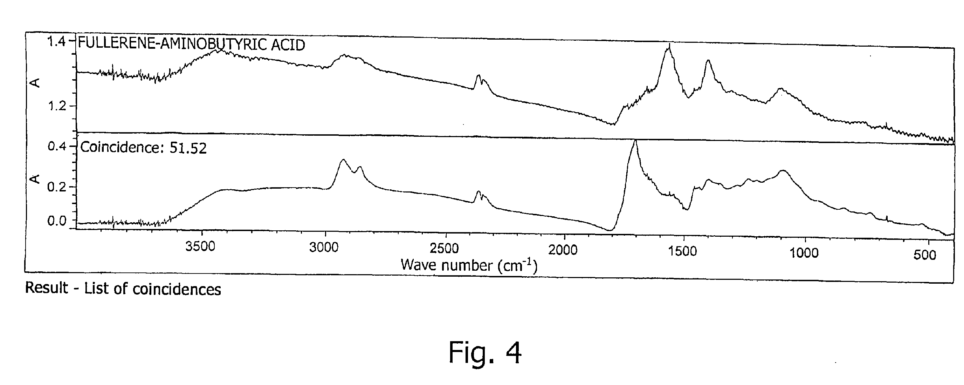

For confirming the presence of the amino acid and fullerene in the claimed compounds, the method of subtracting spectra was employed with subsequent treatment of the results with the aid of the NICOLET EZ OMNIC software, USA (Figs. 2.1, 2.2). The coincidence of the patterns of the IR spectra and absorption bands of the samples of sodium salt of fullerene-polyamino-caproic acid and of fullerene-polyamino-caproic acid obtained by different syntheses was higher than 80%. (The numbers of the syntheses are shown in Fig. 3). The coincidence of the patterns for fullerene-polyamino-butyric and fullerene-polyamino-caproic acids are essentially lower (Fig. 4).

**Example 3.** TLC. Separating compounds of formula (I) by TLC technique

Test solutions of compounds of formula (I) are prepared: of sodium salts of fullerene-polyamino-caproic acid, of fullerene-polyamino-butyric acid and of fullerene-polyamino-octanoic acid in water with the concentration of 1 µg/ml. Fullerene is dissolved in toluene.

10 µl (10 µg) of the test solutions are applied to the starting line of a 10x15 cm Silufol chromatographic plate with a 0.1 mm-thick layer.

The plate is dried in the air for 10 min., then placed into a chamber with a mixture of solvents alcohol benzene : 96% alcohol : water (1:4:1.5) and chromatographed by the ascending technique. When the front of solvents has passed about 10 cm from the starting line, the plate is removed from the chamber and dried in the air for 20 min.

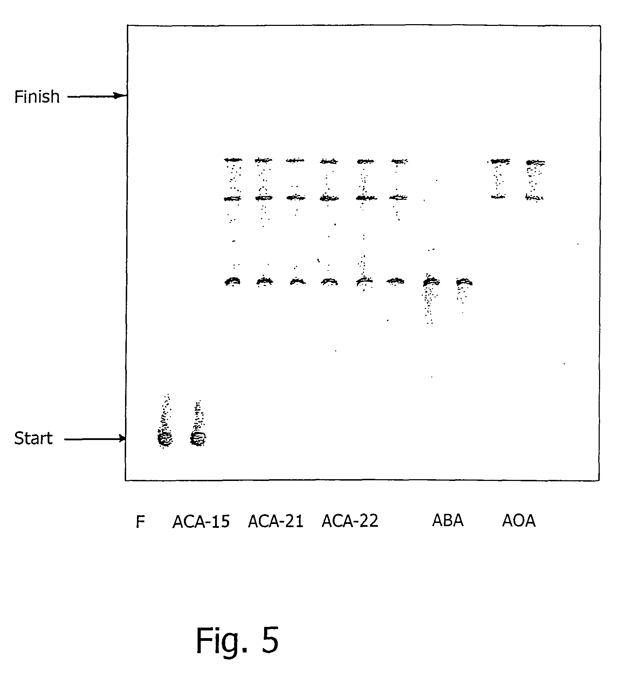

Three spots with R_{f} = 0.82, 0.71, 0.47 are found on the chromatogram: 0.47 for fullerene-polyamino-caproic acid, 0.47 for fullerene-polyamino-butyric acid, and 0.82 for fullerene-polyamino-octanoic acid; fullerene remained at the start (Fig. 5).

TLC. For assessing the content of free amino acid in the preparation.

A test solution of sodium salt of fullerene-polyamino-caproic acid in water with the concentration of 1µg/ml is prepared.

Reference solutions are aqueous solutions of aminocaproic acid (ACA) with the 0.05 µg/ml (5%) and 0.01 µg/ml (1%) concentrations.

10 µl (10 µg) of the test solution and 10 µl of each reference solution are applied to the starting line of a 10x15 cm Silufol chromatographic plate with a 0.1 mm-thick layer.

The plate is dried in the air for 10 min., then placed into a chamber with a mixture of solvents n-butyl alcohol : 96% alcohol : water (2:2:1) and chromatographed by the ascending technique. When the front of solvents has passed about 10 cm from the starting line, the plate is removed from the chamber and dried in the air for 20 min.. then in a drying cabinet at a temperature of 95―100°C for 10 min. The cooled plate is sprayed with a 0.25% solution of ninhydrin in acetone, dried in the air for 5 min., then in a drying cabinet at a temperature of from 95 to 100°C for 5 min.

On the chromatogram of the test solution, in addition to the main spots, the presence of a pink-colored spot is permissible, which does not exceed in the size and color intensity the spot on the chromatogram of the reference solution (not over 5% or 1%, respectively).

The preparation of the 0.25% solution of ninhydrin. 0.25 g of ninhydrin is placed into a 100 ml measuring flask, dissolved in 20 ml of acetone, the volume is brought to the mark and stirred. The solution should be freshly prepared for use.

**Example 4.** Assessing the antiviral activity of compounds of formula (I) on a model of human lymphoblastoid cells with respect to HIV-1.

Cells Reinoculated humans lymphoblastoid cells MT-4 were used. The cell were cultivated in a concentration of 3.0―5.0·10⁵ cells in 1 ml of RPMI 1640 medium with 10% of embryo serum 100 µg/ml of gentamicin. The viability of the cells was determined from the color of a 0.4% solution of trypan blue.

Viruses. As the source of virus the strain of HIV-1_{899A} from the collection of human immunodeficiency viruses at Ivanovskii Institute of Virology, Russian Academy of Medical Sciences, was used.

Retrovir (azidothymidin) produced by GlaxoWellcome (Great Britain) was used as positive control.

Determination of antiviral activity. The Antiviral activity of the preparations was investigated on a model of lymphoblastoid cells in a plastic 24-cell plate. The virus dose was 100 TCD₅₀ (50% tissue cytopathic dose). The cultures were incubated at 37°C in an atmosphere with 5% CO₂ at 98% humidity during 5―7 days till the moment of accounting the results. The activity of the preparation was determined from the inhibition of the virus-induced cytopathic action (CPA) in the cell cultures and the virus antigen level in the culture fluid by immunoenzymatic analysis.

Full protection of the cells from the virus CPA was noted at the dose of the preparation (sodium salt of fullerene-polyamino-propanoic acid) of 1 µg/ml (Table 2).In these concentrations the cytotoxic action of the preparation on the cells was not detected (Tab le 3). The concentration of the preparation was has been determined, at which disappearance of the virus in the culture medium takes place (Table 5). Different schemes of administering the preparation have been studied (Tables 7.1 and 7.2). It has been shown that neither aminocaproic acid nor PEG (Table 8) have such HIV-inhibitory effect.

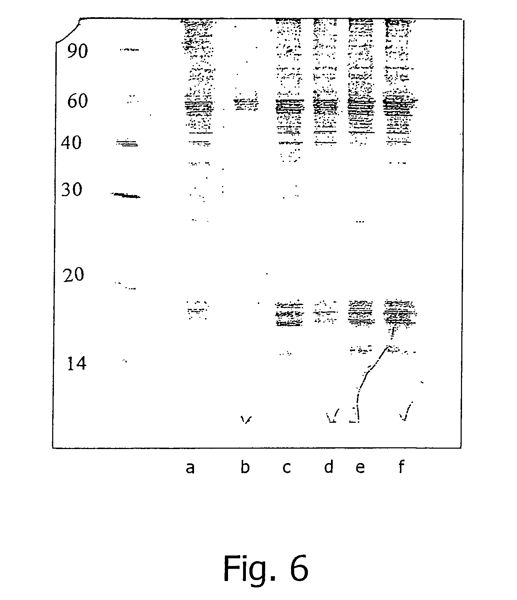

The influence of the compounds of formula (I) on the biosynthetic processes in the culture of HN-1-linfected lymphocytes was studied by the electrophoresis of proteins and nucleic acids in 12.5% PAAG with silver nitrate staining. After cultivation the cells were precipitated by centrifugation and placed into a solution containing Tris, pH 8.0, EDTA, Triton X305, PMSF. The results were assessed from changes of the protein-nucleic profiles in the starting culture, virus-infected and in the presence of the preparation in concentrations of 1, 10 and 100 µg/ml under action on non-infected cells and of 1, 10 µg/ml under action on HIV-infected cells during 96 hours (Fig. 6).

Other fullerene derivatives: sodium salts of fullerene-polyamino-butyric and fullerene-polyamino-octanoic acids have exhibited lower antiviral activity with respect to HIV-1, compared with fullerene-polyamino-caproic acid (Tables 8 and 9).

**Example 5.** Assessing the antiviral activity of compounds of formula (I) on a model of reinoculated cultures of African green monkey kidney cells (VERO) and of human embryo fibroblasts (M-21) with respect to HSV-1.

Cells. Reinoculated cultures of African green monkey kidney cells (VERO) and of human embryo fibroblasts (M-21) obtained from the collection of tissues at Ivanovskii Institute of Virology, Russian Academy of Medical Sciences, were used in the investigation.

Viruses. Herpes simplex virus, type 1, strain L₂, replicated in VERO cells was used in the investigation.

### AZT acyclovir was used as positive control

Investigation of the cytotoxic action. The preparation (sodium salt of fullerene-polyamino-caproic acid) was introduced in concentrations of 500 and 1000 µg/ml into the culture medium of non-infected cells at the stage of monolayer formation. 3-days follow-up of the investigated cultures has not revealed cytodestructive action of the investigated substances.

The protective effect of sodium salt of fullerene-polyamino-caproic acid in concentrations of 1.0, 10 and 50 µg/ml was studied upon introducing the preparation 30 and 60 minutes after the infect ion of the cultures with the virus at the infecting dose of the virus of 100 TCD₅₀. The obtained results have shown that the preparations provided full protection of VERO and M-21 cells from the cytodestructive action of HSV-1 48 hours after the infection of the cell cultures. In this period of the follow-up in control infected cultures which had not undergone the action of the compounds being tested, 100% death of the cells was observed. In the presence of PEG and aminocaproic acid in concentrations of from 10⁻⁴ M to 10⁻² M protection of the cells from HSV was not observed.

The compounds of formula (I), depending on the amino acid radical, have different antiviral activity with respect to HSV-1 (Table 10).

**Example 6.** Assessing the antiviral activity of the compounds of formula (I) on an experimental model of HSV-induced infection in cell cultures.

In our work we used cytopathogenic strain of hepatitis C virus belonging to genotype 1b. The strain was isolated from the blood serum of a female patient suffering from chronic viral hepatitis C, identified as hepatic C virus. Infective doses of HSV equal to 10 TCD of 50/20 µl were used in the investigation.

The investigation was carried out at the laboratory of Ivanovskii Institute of Virology, Russian Academy of Medical Sciences.

Cultures of reinoculated porcine embryo kidney cells (SPEV), highly sensitive to the pathogenic action of HSV, obtained from "Narva" Company (Russia) were employed. These cultures were used in the form of a one day monolayer of cells, grown in 24-cell plastic plates. The cultures of SPEV cells were grown on medium 199 with 10% of fetal calf serum with addition of glutamine and antibiotics (100 U/ml).

For titrating the residual infectiousness of the virus, the same line of porcine embryo kidney cells (SPEV) was used.

Different dilutions of the preparation ― sodium salt of fullerene―polyamino-caproic acid ― from 100 µg/ml and lower on the medium 199 were used in the experiment. For studying the antiviral activity of the preparation, the preparation was introduced in various concentrations into the SPEV cell cultures at the moment of infection and in 24 hours after the infection with hepatitis C virus on the cell in 24-cell plastic culture plates.

The viability of the SPEV cell cultures infected and non-infected with HSV was studied on the 7^{th} day after the infection.

For determining the residual infectious activity of HSV, samples of the culture fluid were taken from the plate cells three days after the treatment of the infected cells with the preparation and titrated on the SPEV cell cultures. The infectious activity of HSV was taken into account from the results of titration on the 6―7^{th} day after the infection, when maximum cytopathogenic action of the virus developed, applying the Rid and Mench formula for calculating the titer of the Hepatitis C virus.

The obtained results have shown that the preparation in a concentration of up to 100 µg/ml does not have cytotoxic properties for the SPEV cell cultures (Table 11).

The preparation in the concentration of 100 µg/ml provided 100% viability of the HSV-infected cells on the 7^{th} day after the infection (Table 12). Under the same conditions Reaferonum induced also 100% survival of the cells.

At the same time, in cell cultures infected with HSV in the dose of 10 TCD₅₀/cell, cytopathogenic phenomena which affected 30―40% of the monolayer developed already by the 4^{th} day, and by the 7^{th} day, as a rule, all the HSV-infected cells died.

The preparation displayed similar activity also in the case when HSV-infected cells were treated with the preparation 24 hours after the infection. However, the preparation has maximum antiviral activity when administered simultaneously with the virus: the virus titers in the SPEV cell cultures lowered by 7.4 lg at the preparation concentration of 100 µg/ml and by 3.0 lg at 50 µg/ml (Table 13).

**Example 7.** The influence of the compounds of formula (I) on the proliferation of reinoculated cells of human epithelial carcinoma Hep 2.

In our experiments we used a 1 day culture of passage 14 Hep 2 cells ― reinoculated cells of human epithelial carcinoma.

The growth medium contained DMEM + glutamine + antibiotics + 5% FBS (fetal bovine serum). The supporting medium was DMEM/needle with a double kit of amino acids and vitamins + glutamine + antibiotics + 10% of cattle serum.

Studying the influence of the preparation on the biosynthetic processes in the cell culture on the example of sodium salt of fullerene-polyamino-caproic acid has shown that the preparation in the studied concentrations of 10, 50 and 100 µg/ml caused pronounced inhibition of the proliferation of Hep 2 cells. The effect of such influence was most pronounced on the seventh day at the concentration of 100 µg/ml: there appeared discontinuities in the monolayer and there were many cell conglomerates in the culture medium.

The monolayer was removed from the glass plate in the 500 µl volume of the medium. The cells were precipitated from 200 µl, resuspended in a lysing buffer; electrophoresis of proteins was carried out in 12.5% PAAG with Kumashi staining.

A reduction in the intensity of protein profiles was observed on the second day and most dramatically on the seventh day at the studied concentrations of the preparation as compared with control. This can be indicative of a reduction in the number of cells because of inhibition of the mitotic activity of cells in the presence of the preparation. On the 7^{th} day of the action of the preparation a reduction in the number of DNA compared with control was observed. Checking for the presence of DNA fragmentation has shown its absence, i.e., apoptosis manifestations were not detected.

For establishing the influence of the preparation on intracellular biosynthetic processes, we have analyzed the synthesis and maturing in the Hep 2 cells of Fas-antigen mRNA. Fas-antigen is a receptor protein of the signal of programmed death of cells ― apoptosis ― and it can exist in two forms: in membrane form and in soluble form. It has been shown that in the presence of the preparation there occurs redistribution of the alternative forms of the Fas-antigen mRNA, this being indicative of the influence of the preparation on the biosynthetic processes in Hep 2 cells on the transcripton level; however, reconstructions indicative of the feasibility of Fas-dependent apoptosis are absent (Fig. 8).

**Example 8.** As examples of dosage forms according to the invention, the following formulations are presented:

Rectal suppositories, the formulation of which comprises compounds of formula (I) ― 0.1 to 200 mg, usually 5 to 20 mg, up to 10% of propylene glycol, and up to 2 g of a lypophilic base.

Capsules: compounds of formula (I) ― 0.1 to 1000 mg, usually 50 to 200 mg, starch or a substitute thereof to the amount required for filling the capsule.

Solutions for injections comprise compounds of formula (I) ― 0.1 to 1.0% of the volume, usually 0.5%, sodium chloride, 850 g; potassium chloride, 30 mg. Water for injections, up to 100 ml.

**Example 9**

Male patient A, born in 1981, earlier did not receive antiviral preparations. Stage 3A of disease since August 2003 was established. There were diagnosed: cytomegalovirus CMV) infection, latent course, candidiasis of the stomatopharinx and of the urinary tracts, herpes simplex, relapsing course. Concomitant diseases: chronic pyelonephritis, relapsing bacterial, chronic hepatitis C with low replication. The initial laboratory analysis data: the number of RNA in plasma is 120000 copies/ml, the virus titer in lymphocytes is equal to 1:8, the quantity of T4-lymphocytes is 59 mm³, the presence of antibodies to HIV-1. The patient volunteered to be treated with the preparation of the invention. The treatment was carried out in accordance with the following schedule: the first month ― administering the preparation (sodium salty of fullerene-polyamino-caproic acid) in the form of 20 mg rectal suppositories, 1 suppository every day during 3 months.

Male patient V, born in 1980. Stage 3A of HIV infection since August 2003 was established. Spread herpetic infection with lesion of the genitalia, inguinal and gluteal areas. Candidiasis of the stomatopharinx. Latent course of CMV infection. Concomitant diseases: Integrative form of chronic hepatitis B. The initial laboratory data: the number of RNA in plasma is 140000 copies/ml, the virus titer in lymphocytes is equal to 1:4, the quantity of T4-lymphocytes is 163 mm³, the T-index is equal to 0.6, the total quantity of lymphocytes is 0.53x10⁻⁶/ml, the presence of antibodies to HIV-1, which makes it possible to draw a conclusion about the disease. The patient volunteered to be treated with the preparation of the invention. The treatment was carried out in accordance with the following schedule: 20 mg rectal suppositories every three days, one suppository a day during 3 months. The data of the clinical analyses of the patients are presented in Table 14.

The use of the preparation of the invention shows an improvement in the condition of the patients.

**Table 1 Solubility of fullerene and its derivatives**

| Compound | | Solvents | | | |
|---|---|---|---|---|---|
| | | Water | Glacial acetic acid | Toluene | o-Dichloro-benzene |
| Fullerene | | No | No | Yes | Yes |
| Fullerene-polyamino-caproic acid | | No | Yes | No | No |
| Sodium salt of fullerene-polyamino-caproic acid | | Yes | Yes | No | No |
| Sodium salt of fullerene-polyamino-butyric acid | | Yes | Yes | No | No |
| Sodium salt of fullerene-polyamino-octanoic acid | | Yes | Yes | No | No |

**Table 2 Investigation of antiviral activity of sodium salt of fullerene-polyamino-caproic acid with respect to human immunodeficiency virus (HIV-1) on a model of lymphoblastoid cells (5^{th} day)**

| Experimental conditions | | Viability of cells, % | Number of cells in 1 ml x 1000 | CPE/syncytia, % |
|---|---|---|---|---|
| Cell control * | | 89 | 1566 | 0 |
| Virus control ** | | 35 | 188 | 100 |
| Preparation, µg/ml | 0.25 | 67 | 1000 | 50 |
| | 0.5 | 71 | 1299 | 25 |
| | 1.0 | 79 | 1466 | 0 |
| | 10 | 87 | 1633 | 0 |
| | 100 | 89 | 1500 | 0 |

| | | | | |
|---|---|---|---|---|
| Note: is non-infected cell control | | | | |
| ** is virus control ― 100 TCA₅₀ | | | | |

**Table 3 Investigation of the cytotoxicity of sodium salt of fullerene-polyamino-caproic salt on a model of human lymphoblastoid cells**

| Experimental conditions | Viability of cells, % | Number of cells in 1 ml x 1000 |
|---|---|---|
| Cell control* | 93 | 966 |
| Preparation, 100 µg/ml | 85 | 866 |

| | | |
|---|---|---|
| Note: * is non-infected cell control | | |

**Table 4 Investigation of the activity of the reference preparation "Retrovir" (0.3 µg/ml) with respect to human immunodeficiency virus on a model of cell cultures**

| Experimental conditions, µg/ml | Viability of cells, % | Number of cells in 1 ml x 1000 | CPE/syncytia, % |
|---|---|---|---|
| Cell control * | 93 | 1300 | 0 |
| Virus control ** | 6 | <<300 | 100 |
| Retrovir | 92 | 1200 | 0 |

| | | | |
|---|---|---|---|
| Note: is non-infected cell control | | | |
| ** is virus control ― 100 TCA₅₀ | | | |

**Table 5 Investigation of the activity of the preparations with respect to human immunodeficiency virus on a model of cell cultures by immunoenzymatic analysis (6^{th} day)**

| Preparation | Concentration, µg/ml | Optical density |
|---|---|---|
| Cell control | ― | 0.073 |
| Virus control | ― | 1.583 |
| Retrovir | 0.3 | 0.055 |
| Sodium salt of fullerene-polyamino-caproic acid | 0.5 | 2.147 |
| | 1.0 | 0.165 |
| | 10 | 0.074 |

| | | |
|---|---|---|
| Note: values > 0.086 are positive for HIV | | |

**Table 6 Investigation of the activity of PEG with respect to human immunodeficiency virus on a model of cell cultures (6^{th} day)**

| Experimental conditions | Toxicity | | HIV-infection | | |
|---|---|---|---|---|---|
| | Viability of cells, % | Number of cells x 10³/ml | Viability of cells, % | Number of cells x 10³/ml | CPE/syncytia, % |
| Cell control | 96 | 1500 | ― | ― | 0 |
| Virus control | ― | ― | 42 | <300 | 100 |
| PEG, 10⁻⁴ M | 92 | 499 | 47 | 366 | 100 |
| PEG, 10⁻³ M | 95 | 460 | 48 | 433 | 100 |
| PEG, 10⁻² M | 57 | <300 | 47 | <300 | 100 |

**Table 7.1 Antiviral activity of sodium salt of fullerene-polyamino-caproic acid, depending on the scheme of introducing the preparation, on a model of human lymphoblastoid cells with respect to HIV-1**

| Experimental conditions | Viability of cells, % | Number of cells in 1 ml x 1000 | CPE/syncytia, % |
|---|---|---|---|
| Cell control * | 96 | 1100 | 0 |
| Virus control ** | 38 | <300 | 100 |
| Preparation, 2 hrs before infecting | 96 | 1100 | 0 |
| Preparation + virus simultaneously | 94 | 1100 | 0 |
| Preparation, 1 hr after infecting | 92 | 1100 | 0 |

**Table 7.2 Antiviral activity of sodium salt of fullerene-polyamino-caproic acid, depending on various schemes of introducing the preparation on a model of human lymphoblastoid cells, with respect to HIV-1**

| Experimental conditions | Exposure | Washing off of cells | 6^{th} day | | | 8^{th} day | |
|---|---|---|---|---|---|---|---|
| | | | Viability of cells, % | Number of cells x 10³ ml | CPE/syncytia, + | Viability of cells, % | CPE/syncytia, + |
| 1 | 2 | 3 | 4 | 5 | 6 | 7 | 8 |
| Cell control | During the entire experiment | No | 91 | 2500 | 0 | 91 | 0 |
| Virus control | During the entire experiment | No | 32 | 400 | +4 | 0 | 4+ |
| Preparation, 1 µg/ml | During the entire experiment | No | 83 | 2300 | 0 | 84 | 0 |
| Preparation, 10 µg/ml | During the entire experiment | No | 91 | 2500 | 0 | 88 | 0 |
| | | | | | | | |
| Preparation, 1 µg/ml | 1 hr | Yes | 83 | 2000 | 0.5+ | 64 | 1.5+ |
| Preparation, 10 µg/ml | 2 hrs | Yes | 84 | 2300 | 0 | 91 | 0 |
| 1 | 2 | 3 | 4 | 5 | 6 | 7 | 8 |
| Preparation, 1µg/ml | 2 hrs | Yes | 88 | 1700 | 0.5+ | 79 | 1+ |

| | | | | | | | |
|---|---|---|---|---|---|---|---|
| Preparation, 10 µg/ml | 2 hrs | Yes | 93 | 1800 | 0 | 91 | 0 |
| Preparation, 1 µg/ml | 24 hrs | Yes | 87 | 2100 | 0.1+ | 89 | 0.5+ |
| Preparation, 10 µg/ml | 24 hrs | Yes | 92 | 2300 | 0 | 91 | 0 |
| | | | | | | | |
| Preparation, 1 µg/ml | 2 hrs of contact | No | 87 | 2200 | 0 | 87 | 0 |
| Preparation, 10 µg/ml | 2 hrs of contact | No | 89 | 2600 | 0 | 89 | 0 |
| | | | | | | | |
| Preparation, 1 µg/ml | 2 hrs of contact | Yes | 69 | 1700 | 2.5+ | 17 | 3.5+ |
| Preparation, 10 µg/ml | 2 hrs of contact | Yes | 74 | 1500 | 1.75+ | 35 | 3.0+ |

**Table 8 Investigation of the cytotoxicity of the preparations of fullerene derivatives on a model of human lymphoblastoid cells with respect to HIV-1**

| Preparation | Concentration, µg/ml | | | |
|---|---|---|---|---|
| | 100 | | 300 | |
| | Viability of cells, % | Number of cells x 10³/ml | Viability of cells, % | Number of cells x 10³/ml |
| ABA* | 76 | 150 | 79 | 60 |
| AOA* | 79 | 150 | 82 | 150 |
| Cell control | 95 | 250 | ― | ― |

**Table 9 Antiviral activity of fullerene derivatives on a model of human lymphoblastoid cells with respect to HIV-1**

| Experimental conditions | Concentration of the preparation, µg/ml | Viability of cells, % | CPE/syncytia, % |
|---|---|---|---|
| Cell control | 0 | 87 | 0 |
| Virus control | 0 | 42 | 100 |
| ABA* | 0.1 | 52 | 25 |
| | 0.5 | 65 | 15 |
| | 1.0 | 71 | 10 |
| | 10.0 | 72 | 0 |
| AOA* | 0.1 | 50 | 25 |
| | 0.5 | 53 | 25 |
| | 1.0 | 57 | 25 |
| | 10.0 | 60 | 0 |

| | | | |
|---|---|---|---|
| AOA ― sodium salt of fullerene-polyamino-octanoic acid | | | |
| ABA ― sodium salt of fullerene-polyamino-butyric acid | | | |

**Table 10 Investigation of the anti-herpetic activity of fullerene derivatives on a model of culture of African green monkey kidney cells (VERO)**

| Preparation | Concentration, µg/ml | Degree of protection from CE virus, % | |
|---|---|---|---|
| ABA | | A* | B* |
| | 0.1 | 0.0 | 33.3 |
| | 10.0 | 33.3 | 33.3 |
| | 25.0 | 100 | 100 |
| | 50.0 | 100 | 100 |
| AOA | 1.0 | 33.3 | 100 |
| | 10.0 | 100 | 100 |

| | | | |
|---|---|---|---|
| Virus: HSV-1, 10 TCD₅₀/ml | | | |
| * Substance contact time with cells: A ― 24 hrs, B ― 48 hrs | | | |

**Table 11 Cytotoxic properties of sodium salt of fullerene-polyamino-caproic acid with respect to the culture of reinoculated porcine embryo kidney cells (SPEV)**

| Cell culture | Percentage of died cells on exposure with different concentrations of the preparation during 48 hours (in µg) | | | | |
|---|---|---|---|---|---|
| | 100 | 50 | 25 | 12.5 | 6.25 |
| SPEV | 0 | 0 | 0 | 0 | 0 |

**Table 12 Viability of HSV-infected of SPEV cell cultures in the presence of sodium salt of fullerene-polyamino-caproic acid on the 7^{th} day after infecting**

| Scheme of introducing the preparation | Percentage of survived SPEV cells | | | | | |
|---|---|---|---|---|---|---|
| | Concentration of the preparation in µg/ml Control | | | | | |
| | 100 | 50 | 25 | 12.5 | 6.25 | |
| At the moment of infecting | 100 | 75 | 0 | 0 | 0 | 0 |
| 24 hours after infecting | 100 | 50 | 0 | 0 | 0 | 0 |

**Table 13 Antiviral activity of sodium salt of fullerene-polyamino-caproic acid, studied on a model of HSV-infected SPEV cell cultures**

| Scheme of introducing the preparation | Titers of hepatitis C virus (lg TCD 50/20 µl) in medium samples taken on the 3^{rd} day after infecting | | | | | |
|---|---|---|---|---|---|---|
| | Concentration of the preparation in µg/ml Control | | | | | |
| | 100 | 50 | 25 | 12.5 | 6.25 | |
| At the moment of infecting | 2.1 | 6.2 | 9.3 | 9.5 | 9.7 | 9.5 |
| 24 hours after infecting | 3.0 | 8.5 | 9.5 | 9.9 | 9.0 | 9.1 |

**Table 14 Investigation of the blood samples of the HIV-1-infected patients treated with the preparation**

| Sample | Date | Virus titer* | PCR, RNA, copies/ml | T4-lymphocytes, mm³ | T8-lymphocytes, mm³ | T-index |
|---|---|---|---|---|---|---|
| Patient A | Before treatment | 8 | 1,200,000 | 59 | 63 | 0.9 |
| | After 1 month | 2 | 72,000 | 355 | 455 | 0.8 |
| | After 2 months | 2 | 43,000 | 234 | 219 | 1.1 |
| | After 3 months | 0 | 44,000 | 381 | 409 | 0.9 |
| Patient B | Before treatment | 4 | 140,000 | 163 | 257 | 0.6 |
| | After 1 month | 128 | 180,000 | 581 | 464 | 1.3 |
| | After 2 months | 2 | 3,500 | 329 | 329 | 1.0 |
| | After 3 months | 1 | 2,000 | 325 | 401 | 0.8 |

| | | | | | | |
|---|---|---|---|---|---|---|
| Note: * value reciprocal to the dilution of sample that gives 50% CPE (or syncytia) in the cell culture | | | | | | |

## Claims

1. An agent for inhibiting membrane virus reproduction, **characterized in that** it comprises a water-soluble compound of fullerene polycarboxylic anions of the general formula
C₆₀Hₙ[NH(CH₂)ₘC(O)O]ₙ,
where C₆₀ is the fullerene core,
NH(CH₂)ₘC(O)O⁻ is the aminocarboxylic anion,
m is an integer, preferably 3 and 5, most preferably 5,
n is an integer from 2 to 12, preferably from 4 to 6, most preferably 6.

2. A method for the production of an agent for inhibiting membrane virus reproduction, **characterized in that** an amino acid in the form of potassium or sodium salt is introduced into an o-dichlorobenzene solution of fullerene, then a solubilizer selected from the group of polyethylene oxides is added: polyethylene glycols with a molecular weight of 150 to 400 and higher, and also dimethyl ethers of polyethylene glycols or 18-crown-6, wherein the amount of the amino acid should be more than 50 times that of fullerene and the synthesis is carried out at a temperature of 60―80°C.

3. A pharmaceutical composition for inhibiting the membrane virus reproduction, **characterized in that** it contains the agent according to claim 1 in an effective amount and pharmaceutically acceptable fillers.

4. A pharmaceutical composition for inhibiting the membrane virus reproduction according to claim 3, **characterized in that** it is prepared in the form of tablets, capsules, a solution for injections, suppositories.

5. A method for inhibiting membrane virus reproduction, **characterized in that** the pharmaceutical composition according to claims 3 and 4 is used for the suppression of viruses when treating diseases caused by HIV, herpes viruses, hepatitis C virus.
